(19) 

**Europäisches Patentamt**
**European Patent Office**
**Office européen des brevets**

(11) **EP 4 600 211 A1**

(12) # EUROPEAN PATENT APPLICATION

(43) Date of publication:
**13.08.2025  Bulletin 2025/33**

(21) Application number: **24167307.8**

(22) Date of filing: **28.03.2024**

(51) International Patent Classification (IPC):
***C01B 33/193*** *(2006.01)*     ***A61K 8/25*** *(2006.01)*
***A61Q 11/00*** *(2006.01)*

(52) Cooperative Patent Classification (CPC):
**C01B 33/193; A61K 8/25; A61Q 11/00;**
**C01B 33/12;** C01P 2004/61; C01P 2006/12;
C01P 2006/22

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority:  **07.02.2024  CN 202410173535**

(71) Applicant: **Jinsanjiang (Zhaoqing) Silicon**
**Material Co., LTD.**
**Zhaoqing, Guangdong (CN)**

(72) Inventors:
• **LIU, Chenglin**
**Zhaoqing, Guangdong (CN)**
• **MI, Shuang**
**Zhaoqing, Guangdong (CN)**
• **LIU, Jing**
**Zhaoqing, Guangdong (CN)**

• **CHEN, Ying**
**Zhaoqing, Guangdong (CN)**
• **ZHENG, Peiwen**
**Zhaoqing, Guangdong (CN)**
• **ZHENG, Yixuan**
**Zhaoqing, Guangdong (CN)**
• **MAO, Xiaowei**
**Zhaoqing, Guangdong (CN)**
• **CHEN, Guanglong**
**Zhaoqing, Guangdong (CN)**
• **CHENG, Dehui**
**Zhaoqing, Guangdong (CN)**
• **HE, Jinli**
**Zhaoqing, Guangdong (CN)**

(74) Representative: **Cabinet Chaillot**
**16/20, avenue de l'Agent Sarre**
**B.P. 74**
**92703 Colombes Cedex (FR)**

(54) **PREPARATION METHOD FOR SILICON DIOXIDE, AND TOOTHPASTE**

(57)    The invention relates to a preparation method for silicon dioxide and belongs to the technical field of silicon dioxides. The invention provides a specific preparation method for silicon dioxide, which controls the temperature, pH and stirring rate in the step of preparing a silicon dioxide solution to exert an influence on the stability of a silicon dioxide solution gel system. The prepared silicon dioxide solution has a low viscosity, and finally prepared silicon dioxide has a high dispersity. The prepared silicon dioxide solution with a low viscosity will not adhere to a reaction tank, thus being easy to discharge. When the silicon dioxide prepared by the preparation method is used for preparing a toothpaste, since the silicon dioxide shows high dispersity due to its large BET specific area, low oil absorption and low viscosity, paste of the prepared toothpaste is not prone to caking and can be rinsed off easily. In addition, the paste of the prepared toothpaste shows high stability in a follow-up test of the viscosity stability of the toothpaste, thus being suitable for long-term storage.

**EP 4 600 211 A1**

# EP 4 600 211 A1

**Description**

**FIELD**

The present invention belongs to the technical field of silicon dioxides, and particularly relates to a preparation method for silicon dioxide, and a toothpaste.

**BACKGROUND**

The principal raw material of common toothpastes is abrasive, which accounts for about 20%-50% of the weight of paste of the toothpastes. The viscosity of the abrasive has a great influence on the viscosity and dispersity of the paste. Silicon dioxide can be used as the abrasive of toothpastes because of its stable physical and chemical properties, acid resistance, alkali resistance, heat resistance, no environmental pollution, good cleaning performance and friction, good adaptability to materials in the toothpastes and high compatibility with fluorine. Chinese Invention Patent Publication No. CN114538452A provides a preparation method for a silicon dioxide wet gel with a narrow particle size distribution, which prepares the silicon dioxide wet gel by a gel method. However, because the viscosity of silicon dioxide is too large (over 3000 cp), the dispersity of paste prepared from the silicon dioxide is low. On the one hand, the toothpastes are prone to caking under high-frequency vibrations of electric toothbrushes, making it difficult to realize uniform cleaning sufficiently; on the other hand, the toothpastes are viscous, being difficult to seep between teeth and difficult to remove, and users have to rinse their mouth repeatedly to wash away the toothpaste, which may also wash away ingredients, beneficial to the mouth, in the toothpastes. In addition, when the traditional gel method is used for preparing silicon dioxide, prepared silicon dioxide adheres into a reaction tank in a gel state during the preparation process of a silicon dioxide solution and is difficult to discharge, causing difficulties to subsequent filtering and washing processes.

**SUMMARY**

A first objective of the invention is to provide a preparation method for silicon dioxide, which can prepare low-viscosity and high-dispersity silicon dioxide.

A second objective of the invention is to provide a toothpaste, using the silicon dioxide prepared above as an abrasive, wherein the silicon dioxide shows high dispersity when applied to the toothpaste, and paste of the prepared toothpaste has a low viscosity and is not too viscous, thus being not prone to caking.

The objectives of the invention can be fulfilled by the following technical solution:

A preparation method for silicon dioxide, comprising the following steps:

providing a sodium silicate solution and a sulfuric acid solution;

preparing a silicon dioxide solution: adding water accounting for 45-60wt% of a total system into a reaction tank, heating the water to 40-60°C, and dropwise adding the sulfuric acid solution to the water and stirring at the same time at a rate of 350-500 rpm until pH reaches 0.5-3.0; then, dropwise adding the sodium silicate solution and the sulfuric acid solution to the water for 1-1.5 hrs until the sodium silicate solution accounts for 30-45wt% of the total system and the sulfuric acid solution accounts for 8-15wt% of the total system, wherein the sodium silicate solution and the sulfuric acid solution are dropwise added at a constant rate, pH in the reaction tank is 0.5-3.0, and a temperature in the reaction tank is 40-60°C; then, aging and stirring at a rate of 350-500 rpm for 30-60min, and after aging and stirring, adjusting the final pH to 2-5 with the sodium silicate solution, thus obtaining the silicon dioxide solution; and

preparing solid silicon dioxide: filter-pressing and washing the silicon dioxide solution with water to obtain a filter cake, drying and crushing the filter cake, and sieving the filter cake to obtain silicon dioxide.

As a preferred solution of the present application, the sodium silicate solution has a concentration of 0.50-1.50 mol/L.

As a preferred solution of the present application, the sodium silicate solution is prepared by: adding solid sodium silicate into a reactor, then adding hot water and steam to liquefy the solid sodium silicate to obtain a concentrated sodium silicate solution, and adding water to the concentrated sodium silicate solution to dilute the concentrated sodium silicate solution to obtain the sodium silicate solution.

As a preferred solution of the present application, the sulfuric acid solution has a concentration of 2.00-8.00 mol/L.

As a preferred solution of the present application, during the preparation process of the solid silicon dioxide, a salt content of the filter cake obtained by filter-pressing and washing is controlled to be less than or equal to 5wt%.

As a preferred solution of the present application, during the preparation process of the solid silicon dioxide, after the filter cake is dried, a loss on drying at 105°C of the silicon dioxide is controlled to be 20-25%.

As a preferred solution of the present application, during the preparation process of the solid silicon dioxide, a particle size of silicon dioxide particles obtained after crushing and sieving of the filter cake is controlled to be 14-19 μm, and a content of 325-mesh undersize particles is greater than or equal to 98%.

A toothpaste using the silicon dioxide described above as an abrasive.

As a preferred solution of the present application, a content of the silicon dioxide in the toothpaste is 1-30%.

The invention has the following beneficial effects:

1. The invention provides a specific preparation method for silicon dioxide, which controls the temperature, pH and stirring rate in the step of preparing a silicon dioxide solution to exert an influence on the stability of a silicon dioxide solution gel system, the prepared silicon dioxide solution has a low viscosity, the oil absorption of finally prepared solid silicon dioxide is lower than 100 g/100 g, the BET specific area is as high as 500~800 $m^2$/g, and the viscosity is as lower as 20-100cp, so the dispersity is high. The prepared silicon dioxide solution has a low viscosity and will not adhere into a reaction tank, thus being easy to discharge from the reaction tank.

2. When the silicon dioxide prepared by the preparation method is used for preparing a toothpaste, because the silicon dioxide shows high dispersity due to its large BET specific area, low oil absorption and low viscosity, paste of the prepared toothpaste is not prone to caking and can be rinsed off easily. In addition, the paste of the prepared toothpaste shows high stability in a follow-up test of the viscosity stability of the toothpaste, thus being suitable for long-term storage.

## DESCRIPTION OF THE EMBODIMENTS

The invention will be described in further detail below in conjunction with specific embodiments.

Embodiment 1

Silicon dioxide was prepared as follows:

A sodium silicate solution was prepared: solid sodium silicate was added into a liquefaction tank, hot water and steam were added into the liquefaction tank, and the solid sodium silicate was liquefied under high temperature and high pressure environment to obtain a concentrated sodium silicate solution; water was added to the concentrated sodium silicate solution to dilute the concentrated sodium silicate solution to obtain 5L of a sodium silicate solution with a concentration of 0.60 mol/L for later use.

A sulfuric acid solution was prepared: water was added to 3 kg of purchased concentrated sulfuric acid to dilute the concentrated sulfuric acid to a concentration of 2.00 mol/L for later use.

A silicon dioxide solution was prepared: 3 kg of water accounting for 45wt% of a total system was added into a reaction tank and heated to 40°C, the sulfuric acid solution was dropwise added into the reaction tank and stirred at a rate of 350 rpm at the same time until the pH reached 0.5-3.0; then, the prepared sodium silicate solution and sulfuric acid solution were dropwise added into the reaction tank for 1 h at a constant rate until the sodium silicate solution accounted for 45wt% of the total system and the sulfuric acid solution accounted for 10wt% of the total system, wherein the pH in the reaction tank was controlled to be 0.5-3.0, and the temperature in the reaction tank was controlled to be 40°C; after the sodium silicate solution and the sulfuric acid solution were added, aging and stirring were carried out at a rate of 350 rpm for 30 min, and then the final pH was adjusted to 2-5 with 0.60 mol/L of the sodium silicate solution, thus obtaining the silicon dioxide solution.

Solid silicon dioxide was prepared: the silicon dioxide solution was filter-pressed and washed with water to obtain a filter cake, the salt content of the filter cake was reduced to be less than or equal to 5wt%, and then the filter cake was dried, wherein the loss on drying at 105°C of the silicon dioxide was controlled to be 20-25%; the dried filter cake was crushed and sieved to obtain silicon dioxide particles, wherein the particle size of the silicon dioxide particles was controlled to be 14-19 μm, and the content of 325-mesh undersize particles was greater than or equal to 98%.

Embodiment 2

Silicon dioxide was prepared as follows:

A sodium silicate solution was prepared: solid sodium silicate was added into a liquefaction tank, hot water and steam were added into the liquefaction tank, and the solid sodium silicate was liquefied under high temperature and high pressure environment to obtain a concentrated sodium silicate solution; water was added to the concentrated sodium silicate solution to dilute the concentrated sodium silicate solution to obtain 5L of a sodium silicate solution with a concentration of 1.20 mol/L for later use.

A sulfuric acid solution was prepared: water was added to 3 kg of purchased concentrated sulfuric acid to dilute the concentrated sulfuric acid to a concentration of 5.00 mol/L for later use.

A silicon dioxide solution was prepared: 3 kg of water accounting for 60wt% of a total system was added into a reaction tank and heated to 50°C, the sulfuric acid solution was dropwise added into the reaction tank and stirred at a rate of 420 rpm at the same time until the pH reached 0.5-3.0; then, the prepared sodium silicate solution and sulfuric acid solution were dropwise added into the reaction tank for 1.2 hrs at a constant rate until the sodium silicate solution accounted for 30wt% of the total system and the sulfuric acid solution accounted for 8wt% of the total system, wherein the pH in the reaction tank was controlled to be 0.5-3.0, and the temperature in the reaction tank was controlled to be 50°C; after the sodium silicate solution and the sulfuric acid solution were added, aging and stirring were carried out at a rate of 420 rpm for 45 min, and then the final pH was adjusted to 2-5 with 1.20 mol/L of the sodium silicate solution, thus obtaining the silicon dioxide solution.

Solid silicon dioxide was prepared: the silicon dioxide solution was filter-pressed and washed with water to obtain a filter cake, the salt content of the filter cake was reduced to be less than or equal to 5wt%, and then the filter cake was dried, wherein the loss on drying at 105°C of the silicon dioxide was controlled to be 20-25%; the dried filter cake was crushed and sieved to obtain silicon dioxide particles, wherein the particle size of the silicon dioxide particles was controlled to be 14-19 $\mu$m, and the content of 325-mesh undersize particles was greater than or equal to 98%.

Embodiment 3

Silicon dioxide was prepared as follows:
A sodium silicate solution was prepared: solid sodium silicate was added into a liquefaction tank, hot water and steam were added into the liquefaction tank, and the solid sodium silicate was liquefied under high temperature and high pressure environment to obtain a concentrated sodium silicate solution; water was added to the concentrated sodium silicate solution to dilute the concentrated sodium silicate solution to obtain 5L of a sodium silicate solution with a concentration of 1.40 mol/L for later use.

A sulfuric acid solution was prepared: water was added to 3 kg of purchased concentrated sulfuric acid to dilute the concentrated sulfuric acid to a concentration of 8.00 mol/L for later use.

A silicon dioxide solution was prepared: 3 kg of water accounting for 45wt% of a total system was added into a reaction tank and heated to 60°C, the sulfuric acid solution was dropwise added into the reaction tank and stirred at a rate of 500 rpm at the same time until the pH reached 0.5-3.0; then, the prepared sodium silicate solution and sulfuric acid solution were dropwise added into the reaction tank for 1.5 hrs at a constant rate until the sodium silicate solution accounted for 40wt% of the total system and the sulfuric acid solution accounted for 15wt% of the total system, wherein the pH in the reaction tank was controlled to be 0.5-3.0, and the temperature in the reaction tank was controlled to be 60°C; after the sodium silicate solution and the sulfuric acid solution were added, aging and stirring were carried out at a rate of 500 rpm for 60 min, and then the final pH was adjusted to 2-5 with 2.50 mol/L of the sodium silicate solution, thus obtaining the silicon dioxide solution.

Solid silicon dioxide was prepared: the silicon dioxide solution was filter-pressed and washed with water to obtain a filter cake, the salt content of the filter cake was reduced to be less than or equal to 5wt%, and then the filter cake was dried, wherein the loss on drying at 105°C of the silicon dioxide was controlled to be 20-25%; the dried filter cake was crushed and sieved to obtain silicon dioxide particles, wherein the particle size of the silicon dioxide particles was controlled to be 14-19 $\mu$m, and the content of 325-mesh undersize particles was greater than or equal to 98%.

Comparative Example 1

Compared with Embodiment 1, this comparative example is identical with Embodiment 1 except that 3 kg of water accounting for 65% of the total system was added into the reaction tank when the silicon dioxide solution was prepared.

Comparative Example 2

Compared with Embodiment 1, this comparative example is identical with Embodiment 1 except that the particle size D50 of the silicon dioxide particles was controlled to be 20-25 $\mu$m when the solid silicon dioxide was prepared.

Comparative Example 3

Compared with Embodiment 1, this comparative example is identical with Embodiment 1 except that the particle size D50 of the silicon dioxide particles was controlled to be 8-12 $\mu$m when the solid silicon dioxide was prepared.

Comparative Example 4

Compared with Embodiment 1, this comparative example is identical with Embodiment 1 except that the loss on drying at 105°C of the silicon dioxide was controlled to be 25-40% when the solid silicon dioxide was prepared.

Comparative Example 5

Compared with Embodiment 1, this comparative example is identical with Embodiment 1 except that the loss on drying at 105°C of the silicon dioxide was controlled to be 10-15% when the solid silicon dioxide was prepared.

The silicon dioxide prepared in Embodiments 1-3 and Comparative Examples 1-5 was tested as follows:
Test 1: the properties of silicon dioxide powder prepared in Embodiments 1-3 and Comparative Examples 1-5 were tested:

(1) Method for measuring the viscosity of the silicon dioxide:
40 g of the silicon dioxide prepared in Embodiments 1-3 and Comparative Examples 1-5 was weighed and placed in a 300 mL plastic cup, 200 g of distilled water was added into the plastic cup, and the silicon dioxide and the distilled water were evenly stirred by means of a glass rod and then stirred for 10 min by means of a stirrer with a rotational speed of 25 Hz. After stirring, the viscosity was measured by means of a DV-II+pro viscosimeter. Obtained results are shown in Table 1.

(2) Method for measuring the oil absorption of the silicon dioxide:
1.00 g of the silicon dioxide prepared in Embodiments 1-3 and Comparative Examples 1-5 was weighed and placed on a glass plate, and linseed oil was dropwise added to the silicon dioxide by means of a micro-burette and continuously stirred by means of a spatula to gradually thicken samples until the samples were completely thickened and formed paste, with no viscous matter and silicon dioxide power being left on the glass plate.
The oil absorption is calculated by the following formula, and obtained results are shown in Table 1:

$$X = \frac{m_2}{m_1} \times 100$$

where,

X - oil absorption (g/100g);

$m_1$ - mass of sample (g);

$m_2$ -mass of linseed oil consumption (g).

(3) Method for measuring the BET specific area of the silicon dioxide:
The BET specific area of the silicon dioxide prepared in Embodiments 1-3 and Comparative Examples 1-5 was measured by means of a JW-BK112 static nitrogen absorption instrument respectively. Obtained results are shown in Table 1.

(4) Method for measuring the particle size distribution of powder:
The particle size distribution was measured by means of a BT-9300H laser particle size distribution instrument. Obtained results are shown in Table 1.

(5) Method for measuring the loss on drying at 105°C of the silicon dioxide:
The volatile content of the silicon dioxide was measured according to the measurement method of "5.4 Volatiles" in QB/T 2346-2015.

(6) Method for measuring the content of 325-mesh undersize particles of the silicon dioxide:
The content of 325-mesh undersize particles of the silicon dioxide was measured according to the measurement method of "5.3 Undersize particles" in QB/T 2346-2015.

(7) Method for measuring copper consumption of the silicon dioxide:

According to Article ⟨⟨Study on Copper Consumption of Silicon Dioxide for Toothpaste⟩⟩, a copper sheet was cleaned with distilled water and dried, and the copper sheet was marked and weighed; the copper sheet was fixed in a locking slot of a material pool, and the silicon dioxide powder prepared in Embodiments 1-3 and Comparative Examples 1-5 was accurately dispersed in sorbitol, evenly stirred and then poured into the material pool to drown the copper sheet; the test was ended after the copper sheet was rubbed repeatedly 10,000 times, the copper sheet was cleaned, dried and then weighed, and the mass difference of the copper sheet before and after rubbing was the copper consumption. Obtained results are shown in Table 1.

Table 1

| Group | Oil absorption g/100g | Particle size D50 $\mu$m | Content of undersize particles % | loss on drying at 105°C % | Viscosity cp | Copper consumption mg | BET specific area m$^2$/g |
|---|---|---|---|---|---|---|---|
| Embodiment 1 | 50 | 17 | 98 | 20 | 48 | 0.6 | 658 |
| Embodiment 2 | 55 | 14 | 98 | 20 | 85 | 0.5 | 761 |
| Embodiment 3 | 44 | 14 | 98 | 24 | 23 | 0.8 | 622 |
| Comparative Example 1 | 123 | 17 | 98 | 20 | 237 | 0.3 | 915 |
| Comparative Example 2 | 34 | 24 | 98 | 19 | 18 | 1.2 | 471 |
| Comparative Example 3 | 67 | 9 | 98 | 20 | 305 | 0.4 | 1062 |
| Comparative Example 4 | 40 | 17 | 98 | 35 | 15 | 0.8 | 331 |
| Comparative Example 5 | 83 | 16 | 98 | 12 | 546 | 0.4 | 962 |

It can be seen from Table 1 that the oil absorption of the silicon dioxide prepared in the embodiments of the invention is 40-60 g/100 g, the viscosity is 23-85 cp, and the BET specific area is 622-761 m$^2$/g, indicating that the silicon dioxide has a larger specific area, a lower oil absorption and a lower viscosity; and the particle size D50 of the silicon dioxide is 14-17$\mu$m, the loss on drying at 105°C is 20-24%, and the copper consumption is 0.5-0.8 mg, thus satisfying subsequent toothpaste preparation requirements. The oil absorption and viscosity of the silicon dioxide in Comparative Example 1 are remarkably improved; the particle size D50 of the silicon dioxide in Comparative Example 2 is 24 $\mu$m, and the particle size D50 of the silicon dioxide in Comparative Example 3 is 9 $\mu$m, both out of the range of 14-19 $\mu$m. The silicon dioxide in the Comparative Example 2 has higher copper consumption and a lower BET specific area, and the silicon dioxide in Comparative Example 3 has a higher viscosity, so the silicon dioxide in the Comparative Example 2 and the silicon dioxide in Comparative Example 3 are not suitable for preparing a toothpaste. The loss on drying at 105°C of the silicon dioxide in Comparative Example 4 is 35%, and the loss on drying at 105°C of the silicon dioxide in Comparative Example 5 is 12%, both out of the range of 20-25%, the silicon dioxide in Comparative Example 4 has a lower BET specific area, and the silicon dioxide in Comparative Example 5 has a higher viscosity, so the silicon dioxide in Comparative Example 4 and the silicon dioxide in Comparative Example 5 are not suitable for preparing a toothpaste.

The silicon dioxide prepared in Embodiments 1-3 and Comparative Examples 1-5 was used for preparing toothpastes according to the ingredients in Table 2, and the toothpastes prepared are shown in Table 3.

Table 2

| Ingredients of toothpaste | Mass ratio / % |
|---|---|
| Sorbitol | 50 |
| Carboxymethylcellulose | 1.0 |
| Sodium saccharin | 0.2 |
| Lauryl sodium sulfate | 2.0 |
| Spearmint oil | 1.0 |
| Sodium benzoate | 0.2 |
| Water | 20.4 |
| Titanium dioxide | 0.2 |

(continued)

| Ingredients of toothpaste | Mass ratio / % |
|---|---|
| Silicon dioxide | 25.0 |
| Total | 100 |

Table 3

| Silicon dioxide used | Embodiment 1 | Embodiment 2 | Embodiment 3 | Comparative Example 1 | Comparative Example 2 | Comparative Example 3 | Comparative Example 4 | Comparative Example 5 |
|---|---|---|---|---|---|---|---|---|
| Toothpaste | Embodiment 4 | Embodiment 5 | Embodiment 6 | Comparative Example 6 | Comparative Example 7 | Comparative Example 8 | Comparative Example 9 | Comparative Example 10 |

The toothpaste prepared using the silicon dioxide prepared in Embodiments 1-3 and Comparative Examples 1-5 are shown in Table 3. The toothpaste in Embodiments 4-6 and Comparative Examples 6-10 were tested as follows:

Test 2: test of the viscosity of the toothpastes

50 g of the toothpaste was weighed and placed in a 300 ml plastic cup, the toothpaste was maintained in a 25°C environment until the temperature of the toothpaste reached 25°C, and the viscosity of the toothpaste was measured by means of a DV-II+pro viscometer, wherein the viscosity is the viscosity of the toothpaste tested on the day when the toothpaste was prepared. Obtained results are shown in Table 4.

Test 3: method for testing the relaxation time of the toothpastes

The relaxation time indicates the dispersity of the toothpastes, and the shorter the relaxation time, the better the dispersity of the toothpastes.

A sample was maintained at a constant temperature for 1 h by means of a dry thermostat, and then the relaxation time of the toothpastes was tested by means of a LISICO LS-1 dispersion stability analysis instrument. Obtained results are shown in Table 4.

Test 4: test of the properties of the toothpastes in use

40 volunteers were selected and randomly divided into 8 groups to test the appearance and brushing experience of the eight toothpastes. During the brushing test, the taste and dispersity of the toothpastes were scored: 3 scores for an extremely fresh taste, 2 scores for a fresh taste, and 1 score for a common taste; 3 scores for an extremely good dispersity, 2 scores for a good dispersity, and 1 score for a moderate dispersity; and average scores were calculated. Obtained results are shown in Table 4.

Table 4

| Number of samples | Viscosity Wcp | Relaxation time ms | Appearance | Brushing experience test | |
|---|---|---|---|---|---|
| | | | | Taste | Dispersity |
| Embodiment 4 | 23 | 36.43 | The paste was uniform and free of foreign matter, and had a moderate viscosity | 3 | 3 |
| Embodiment 5 | 15 | 33.51 | The paste was uniform and free of foreign matter, and had a relatively low viscosity | 2 | 3 |
| Embodiment 6 | 19 | 35.76 | The paste was uniform and free of foreign matter, and had a relatively low viscosity | 2 | 3 |
| Comparative Example 6 | 63 | 42.64 | The paste was uniform and free of foreign matter, and had a relatively high viscosity | 2 | 2 |
| Comparative Example 7 | 13 | 30.15 | The paste was uniform and free of foreign matter, and had an excessively low viscosity | 2 | 3 |
| Comparative Example 8 | 71 | 44.01 | The paste was uniform and free of foreign matter, and had a high viscosity | 1 | 1 |
| Comparative Example 9 | 10 | 28.12 | The paste was uniform and free of foreign matter, and had an excessively low viscosity | 2 | 3 |
| Comparative Example 10 | 80 | 46.22 | The paste was uniform and free of foreign matter, and had an excessively high viscosity | 2 | 1 |

It can be known, from Table 4, that the toothpastes in Embodiments 4-6 of the invention have a viscosity of 15-23 cp and a relaxation time of 33-37 ms and show the characteristics of uniform paste and viscosity in use. The paste of the toothpaste in Comparative Example 6 has an excessively high viscosity and is thick, the paste of the toothpaste in Comparative Example 7 has an excessively low viscosity and is too thin, the paste of the toothpaste in Comparative Example 8 has an excessively high viscosity and is thick, the paste of the toothpaste in Comparative Example 9 has an excessively low viscosity and is too thin, and the paste of the toothpaste in Comparative Example 10 has an excessively high viscosity and is too thick. It can be seen that the silicon dioxide prepared in Embodiments 1-3 shows an appropriate viscosity and dispersity in the paste of the toothpaste.

The toothpaste in Embodiment 4 was tested as follows:

Test 5: follow-up test of the viscosity stability of the toothpaste

A 90 day's test was carried out on the toothpaste in Embodiment 4. Specific viscosity data are shown in Table 5.

Table 5

| Time | Temperature/°C | Viscosity/wcp |
|------|------|------|
| Day 1 | -10 | 23.0 |
| | 25 | 23.2 |
| | 40 | 25.0 |
| Day 7 | -10 | 23.0 |
| | 25 | 26.3 |
| | 40 | 32.9 |
| Day 30 | -10 | 26.9 |
| | 25 | 30.8 |
| | 40 | 37.8 |
| Day 60 | -10 | 28.8 |
| | 25 | 32.0 |
| | 40 | 39.5 |
| Day 90 | -10 | 29.2 |
| | 25 | 32.4 |
| | 40 | 39.8 |

It can be known, from Table 5, that the viscosity of the paste of the toothpaste in Embodiment merely changes from 23.0 wcp to 29.2 wcp at a temperature of -10°C, merely changes from 23.2 wcp to 32.4 wcp at a temperature of 25°C, and merely changes from 25.0 wcp to 39.8 wcp at a temperature of 40°C, indicating that the paste of the toothpaste has a stable viscosity and is suitable for long-term storage.

The above description is merely for explaining the embodiments of the invention and is not intended to limit the patent protection scope of the invention. All inessential variations or substitutions made by those skilled in the art based on the invention should still fall within the protection scope of the invention.

**Claims**

1. A preparation method for silicon dioxide, **characterized by** comprising the following steps:

providing a sodium silicate solution and a sulfuric acid solution;
preparing a silicon dioxide solution: adding water accounting for 45-60wt% of a total system into a reaction tank, heating the water to 40-60°C, and dropwise adding the sulfuric acid solution to the water and stirring at the same time at a rate of 350-500 rpm until pH reaches 0.5-3.0; then, dropwise adding the sodium silicate solution and the sulfuric acid solution to the water for 1-1.5 hrs until the sodium silicate solution accounts for 30-45wt% of the total system and the sulfuric acid solution accounts for 8-15wt% of the total system, wherein the sodium silicate solution and the sulfuric acid solution are dropwise added at a constant rate, pH in the reaction tank is 0.5-3.0, and a temperature in the reaction tank is 40-60°C; then, aging and stirring at a rate of 350-500 rpm for 30-60min, and after aging and stirring, adjusting the final pH to 2-5 with the sodium silicate solution, thus obtaining the silicon dioxide solution; and
preparing solid silicon dioxide: filter-pressing and washing the silicon dioxide solution with water to obtain a filter cake, drying and crushing the filter cake, and sieving the filter cake to obtain silicon dioxide.

2. The preparation method according to Claim 1, **characterized in that** the sodium silicate solution has a concentration of 0.50-1.50 mol/L.

3. The preparation method according to Claim 1, **characterized in that** the sodium silicate solution is prepared by: adding solid sodium silicate into a reactor, then adding hot water and steam to liquefy the solid sodium silicate to obtain a concentrated sodium silicate solution, and adding water to the concentrated sodium silicate solution to dilute the concentrated sodium silicate solution to obtain the sodium silicate solution.

4. The preparation method according to Claim 1, **characterized in that** the sulfuric acid solution has a concentration of 2.00-8.00 mol/L.

5. The preparation method according to Claim 1, **characterized in that** during the preparation process of the solid silicon dioxide, a salt content of the filter cake obtained by filter-pressing and washing is controlled to be less than or equal to 5wt%.

6. The preparation method according to Claim 1, **characterized in that** during the preparation process of the solid silicon dioxide, after the filter cake is dried, a loss on drying at 105°C of the silicon dioxide is controlled to be 20-25%.

7. The preparation method according to Claim 1, **characterized in that** during the preparation process of the solid silicon dioxide, a particle size of silicon dioxide particles obtained after crushing and sieving of the filter cake is controlled to be 14-19 $\mu$m, and a content of 325-mesh undersize particles is greater than or equal to 98%.

8. A toothpaste, **characterized by** using the silicon dioxide according to any one of Claims 1-7 as an abrasive.

9. The toothpaste according to Claim 8, **characterized in that** a content of the silicon dioxide in the toothpaste is 1-30%.

Europäisches
Patentamt

European
Patent Office

Office européen
des brevets

# EUROPEAN SEARCH REPORT

**Application Number**

EP 24 16 7307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 538 452 B (JINSANJIANG ZHAOQING SILICON MAT SHARE LIMITED COMPANY ET AL.) 25 October 2022 (2022-10-25) | 8 | INV. C01B33/193 A61K8/25 A61Q11/00 |
| Y | * the whole document * | 1-7 | |
| X | US 2021/230008 A1 (FERAL-MARTIN CÉDRIC [FR]) 29 July 2021 (2021-07-29) | 8,9 | |
| Y | * paragraphs [0029] - [0099] * * examples 1-3 * | 1-7 | |
| X | JP 4 515761 B2 (RHODIA CHIMIE) 4 August 2010 (2010-08-04) | 8 | |
| Y | * the whole document * | 1-7 | |
| X | CN 111 392 739 B (GUANGZHOU FEIXUE MATERIAL TECH CO LTD ET AL.) 1 December 2020 (2020-12-01) | 8 | |
| Y | * the whole document * | 1-7 | |
| X | CN 112 456 505 B (GUANGZHOU FEIXUE MATERIAL TECH CO LTD) 9 July 2021 (2021-07-09) | 8 | TECHNICAL FIELDS SEARCHED (IPC) |
| Y | * the whole document * | 1-7 | C01B A61Q A61K |

The present search report has been drawn up for all claims

| Place of search | Date of completion of the search | Examiner |
|---|---|---|
| The Hague | 6 September 2024 | Marino, Emanuela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 7307

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2024

| Patent document cited in search report | | Publication date | Patent family member(s) | | Publication date |
|---|---|---|---|---|---|
| CN 114538452 | B | 25-10-2022 | NONE | | |
| US 2021230008 | A1 | 29-07-2021 | BR 112020025240 | A2 | 09-03-2021 |
| | | | BR 122024001403 | A2 | 27-02-2024 |
| | | | CN 112334121 | A | 05-02-2021 |
| | | | CN 117865167 | A | 12-04-2024 |
| | | | EP 3810068 | A1 | 28-04-2021 |
| | | | JP 2021528349 | A | 21-10-2021 |
| | | | KR 20210021520 | A | 26-02-2021 |
| | | | US 2021230008 | A1 | 29-07-2021 |
| | | | WO 2019243165 | A1 | 26-12-2019 |
| JP 4515761 | B2 | 04-08-2010 | AU 2002341047 | B2 | 09-11-2006 |
| | | | BR 0211703 | A | 28-09-2004 |
| | | | CA 2457769 | A1 | 27-02-2003 |
| | | | CA 2729228 | A1 | 27-02-2003 |
| | | | CN 1541186 | A | 27-10-2004 |
| | | | CN 101412515 | A | 22-04-2009 |
| | | | DK 1419106 | T3 | 30-01-2017 |
| | | | EP 1419106 | A1 | 19-05-2004 |
| | | | EP 2325141 | A1 | 25-05-2011 |
| | | | EP 3078634 | A1 | 12-10-2016 |
| | | | ES 2610590 | T3 | 28-04-2017 |
| | | | JP 4515761 | B2 | 04-08-2010 |
| | | | JP 2005500238 | A | 06-01-2005 |
| | | | KR 20040030070 | A | 08-04-2004 |
| | | | MX PA04001133 | A | 20-05-2004 |
| | | | PT 1419106 | T | 27-12-2016 |
| | | | TW I247725 | B | 21-01-2006 |
| | | | US 2005032965 | A1 | 10-02-2005 |
| | | | US 2010221541 | A1 | 02-09-2010 |
| | | | US 2011263784 | A1 | 27-10-2011 |
| | | | WO 03016215 | A1 | 27-02-2003 |
| CN 111392739 | B | 01-12-2020 | NONE | | |
| CN 112456505 | B | 09-07-2021 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 114538452 A